(19) Europäisches Patentamt
European Patent Office
Office européen des brevets

(11) **EP 3 299 976 A1**

(12) **EUROPEAN PATENT APPLICATION**
published in accordance with Art. 153(4) EPC

(43) Date of publication:
**28.03.2018 Bulletin 2018/13**

(51) Int Cl.:
***G06F 19/24*** (2011.01)

(21) Application number: **16899247.7**

(22) Date of filing: **17.11.2016**

(86) International application number:
**PCT/CN2016/106255**

(87) International publication number:
**WO 2017/181665 (26.10.2017 Gazette 2017/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **20.04.2016 CN 201610246971**

(71) Applicant: **Soochow University**
**Suzhou, Jiangsu 215123 (CN)**

(72) Inventors:
• **ZHANG, Li**
**Suzhou**
**Jiangsu 215123 (CN)**
• **HUANG, Xiaojuan**
**Suzhou**
**Jiangsu 215123 (CN)**
• **WANG, Bangjun**
**Suzhou**
**Jiangsu 215123 (CN)**
• **ZHANG, Zhao**
**Suzhou**
**Jiangsu 215123 (CN)**
• **LI, Fanzhang**
**Suzhou**
**Jiangsu 215123 (CN)**

(74) Representative: **zacco Dr. Peters & Partner**
**Am Wall 187-189**
**28195 Bremen (DE)**

(54) **GENE EXPRESSION DATA CLASSIFICATION METHOD AND CLASSIFICATION SYSTEM**

(57) A gene expression data classification method and a gene expression data classification system are provided. With the gene expression data classification method, a gene feature data set is acquired, and then the gene feature data set is clustered using a clustering algorithm to obtain clustering sets, the number of which is a first preset parameter, the clustering sets are processed to obtain a second sample matrix, a second training set and a feature index set, to reduce dimensionality of gene expression data. In this way, redundancy among the gene expression data is reduced, thereby greatly reducing calculation resources and calculation time consumed in a subsequent process of performing feature selection on the second training set. Also, a few calculation resources and a little calculation time are consumed in a case of clustering the gene feature data set by using the clustering algorithm, therefore, a few calculation resources and a little calculation time are consumed by classifying the gene expression data to be measured with the gene expression data classification method.

Figure 1

## Description

The present disclosure claims the priority to Chinese Patent Application No. 201610246971.3, titled "GENE EXPRESSION DATA CLASSIFICATION METHOD AND CLASSIFICATION SYSTEM", filed on April 20, 2016 with the Chinese State Intellectual Property Office, the content of which is incorporated herein by reference.

## TECHNICAL FIELD

The present disclosure relates to the technical field of gene classification, and in particular to a gene expression data classification method and a gene expression data classification system.

## BACKGROUND

Gene expression data with tens of thousands of dimensions can be simultaneously measured with DNA microarray technology, and the gene expression data can help researchers to study nature of organisms. However, only a small part of a large amount of gene expression data is a research object of the researchers. Taking research on cancer genes as example, the number of samples of cancer gene expression data is usually smaller than one hundred, and it consumes a lot of calculation resources and calculation time to classify a large amount of gene expression data as the cancer genes and other genes.

Some researchers apply a support vector machine recursive feature elimination (SVM-RFE) algorithm to automatically eliminate redundant genes (that is, genes which do not contribute to gene classification) in the large amount of gene expression data, so as to find target genes from the large amount of the gene expression data.

However, feature selection processing is to be performed on the large amount of the gene expression data in the SVM-RFE algorithm, which consumes a lot of calculation resources and a lot of calculation time.

## SUMMARY

In order to solve the above technical problem, a gene expression data classification method and a gene expression data classification system are provided according to the present disclosure, to solve the problem that classification for gene expression data consumes a lot of calculation resources and a lot of calculation time.

To solve the above technical problem, the following technical solutions are provided according to embodiments of the present disclosure.

A gene expression data classification method is provided, which includes:

acquiring a first training set and generating a gene feature data set based on the first training set, where the first training set includes gene expression data;

clustering the gene feature data set by using a clustering algorithm to obtain clustering sets, the number of which is a first preset parameter, where each of the clustering sets includes one clustering center;

generating a second sample matrix based on representative genes of all the clustering sets, where the representative gene is one gene in each of the clustering sets;

processing the second sample matrix to obtain a second training set;

generating a feature index set corresponding to the second training set;

ranking the second training set based on features to obtain a sequential feature index set corresponding to the ranked second training set;

selecting, from the sequential feature index set, consecutive features from a first feature, the number of which is a second preset parameter, to constitute a third training set;

modeling the third training set to obtain a model function; and

classifying gene expression data to be measured based on the feature index set, the sequential feature index set and the model function, to obtain a classification result of the gene expression data to be measured.

Preferably, setting of the first preset parameter may include:

processing the gene feature data set with an N-fold cross validation method, and determining a value corresponding to a maximum recognition rate as the first preset parameter, where N is 5, 10 or 20.

Preferably, the clustering the gene feature data set by using the clustering algorithm, to obtain the clustering sets, the number of which being the first preset parameter, and each of the clustering sets including one clustering center may include:

clustering the gene feature data set by using a K-means clustering algorithm, to obtain the clustering sets, the number of which is the first preset parameter, where each of the clustering sets includes one clustering center.

Preferably, the representative gene may be generated according to an equation $\overline{\mathbf{g}}_k = \min\limits_{\mathbf{g}_i \in G_k} \left\| \mathbf{g}_i - \mathbf{m}_k \right\|_2^2$, $k = 1$, ..., $K$; where $G_k$ represents a k-th clustering set, $\overline{\mathbf{g}}_k$ represents the representative gene of the k-th clustering set, $m_k$ represents a k-th clustering center, K represents the first preset parameter, and $g_i$ represents the gene expression data of the clustering set; and

where the second sample matrix is represented as $\mathbf{X}' = [\overline{\mathbf{g}}_1, \cdots, \overline{\mathbf{g}}_k]^T \in R^{K \times N}$, R represents a set of real numbers, and N represents the total number of samples in the first training set.

Preferably, the classifying the gene expression data to be measured based on the feature index set, the sequential feature index set and the model function, to obtain the classification result of the gene expression data to be measured may include:

performing, based on the feature index set, feature selection on the gene expression data to be measured, to obtain a sample after first feature selection;
selecting, from the sample after the first feature selection based on the sequential feature index set, consecutive features from a first feature, the number of which is the second preset parameter, to constitute a sample after second feature selection; and
inputting the sample after the second feature selection into the model function to obtain an output result of the model function, and obtaining, based on the output result, the classification result of the gene expression data to be measured.

A gene expression data classification system is provided, which includes:

a feature selecting module configured to: acquire a first training set and generate a gene feature data set based on the first training set, where the first training set includes gene expression data; cluster the gene feature data set by using a clustering algorithm to obtain clustering sets, the number of which is a first preset parameter, where each of the clustering sets includes one clustering center; generate a second sample matrix based on representative genes of all the clustering sets, where the representative gene is one gene in each of the clustering sets; process the second sample matrix to obtain a second training set; generate a feature index set corresponding to the second training set; rank the second training set based on features to obtain a sequential feature index set corresponding to the ranked second training set; and select, from the sequential feature index set, consecutive features from a first feature, the number of which is a second preset parameter, to constitute a third training set;

a training module configured to model the third training set to obtain a model function; and

a diagnosing module configured to classify gene expression data to be measured based on the feature index set, the sequential feature index set and the model function, to obtain a classification result of the gene expression data to be measured.

Preferably, the feature selecting module may include:

a preprocessing unit configured to acquire a first training set of gene samples, preprocess the first training set to generate a first sample matrix, and generate the gene feature data set based on the sample matrix;

a first feature selecting unit configured to: process the gene feature data set with an N-fold cross validation method, and determine a value corresponding to a maximum recognition rate as the first preset parameter, where N is 5, 10 or 20; cluster the gene feature data set by using a K-means clustering algorithm, to obtain clustering sets, the number of which is the first preset parameter, where each of the clustering sets includes one clustering center; select one gene from each of the clustering sets as the representative gene of the clustering set, and generate the second sample matrix based on the representative genes of all the clustering sets; and process the second sample matrix to obtain the second training set, and generate the feature index set corresponding to the second training set; and

a second feature selecting unit configured to rank the second training set based on features to obtain the sequential feature index set, determine the second preset parameter as the number of reserved features, and select, from the sequential feature index set, consecutive features from a first feature, the number of which is the second preset parameter, to constitute the third training set.

Preferably, the first feature selecting unit may be configured to: process the gene feature data set with the N-fold cross validation method, and determine the value corresponding to the maximum recognition rate as the first preset parameter, where N is 5, 10 or 20; cluster the gene feature data set by using the K-means clustering algorithm, to obtain clustering centers and clustering sets, with each of the numbers of the clustering centers and the clustering sets being the first preset parameter; select one gene from each of the clustering sets as the representative gene of the clustering set, generate the second sample matrix based on the representative genes of all the clustering sets, and select each column $\mathbf{x}'_i$ of the second sample matrix to constitute the second training set $\{\mathbf{x}'_i, y_i\}_{i=1}^N$, where $\mathbf{x}'_i \in R^K$.

Preferably, the representative gene may be generated according to an equation $\overline{\mathbf{g}}_k = \min\limits_{\mathbf{g}_i \in G_k} \left\| \mathbf{g}_i - \mathbf{m}_k \right\|_2^2$, $k = 1$, ..., $K$, where $G_k$ represents a k-th clustering set, $\overline{\mathbf{g}}_k$ represents the representative gene of the k-th clustering set, $m_k$ represents a k-th clustering center, K represents the first preset parameter, and $g_i$ represents the gene expression data of the clustering set; and

where the second sample matrix is represented as $\mathbf{X}' = [\overline{\mathbf{g}}_1, \cdots, \overline{\mathbf{g}}_k]^T \in R^{K \times N}$, R represents a set of real numbers, and N represents the total number of samples in the generated first training set.

Preferably, the diagnosing module may include:

a first selecting unit configured to perform, based on the feature index set, feature selection on gene expression data to be measured, to obtain a sample after first feature selection;

a second selecting unit configured to select, from the sample after the first feature selection based on the sequential feature index set, consecutive features from a first feature, the number of which is the second preset parameter, to constitute a sample after second feature selection; and

a diagnosing unit configured to input the sample after the second feature selection into the model function to obtain an output result of the model function, and obtain, based on the output result, the classification result of the gene expression data to be measured.

It can be seen from the above technical solutions that the gene expression data classification method and the gene expression data classification system are provided according to the embodiments of the present disclosure. With the gene expression data classification method, the gene feature data set is obtained; the gene feature data set is clustered using the clustering algorithm to obtain the clustering sets, the number of which is the first preset parameter; and the clustering sets are processed to obtain the second sample matrix, the second training set and the feature index set, to reduce dimensionality of the gene expression data, and thus redundancy among the gene expression data is reduced, thereby greatly reducing calculation resources and calculation time consumed in the subsequent process of performing feature selection on the second training set. Also, a few calculation resources and a little calculation time are consumed in a case of clustering the gene feature data set by using the clustering algorithm, therefore, a few calculation resources and a little calculation time are consumed in a case of classifying the gene expression data to be measured with the gene expression data classification method.

**BRIEF DESCRIPTION OF THE DRAWINGS**

In order to clearly illustrate the technical solutions according to the embodiments of the present disclosure or the conventional technology, the drawings required in description of the embodiments or the conventional technology are introduced below briefly. Apparently, the drawings in the following description illustrate only some embodiments of the present disclosure, and other drawings may be obtained by those skilled in the art based on the provided drawings without creative efforts.

Figure 1 is a schematic flowchart of a gene expression data classification method according to an embodiment of the present disclosure;

Figure 2 is a schematic flowchart of a gene expression data classification method according to another embodiment of the present disclosure;

Figure 3 is a schematic structural diagram of a gene expression data classification system according to an embodiment of the present disclosure;

Figure 4 is a schematic structural diagram of a feature selecting module according to an embodiment of the present disclosure; and

Figure 5 is a schematic structural diagram of a diagnosing module according to an embodiment of the present disclosure.

## DETAILED DESCRIPTION OF THE EMBODIMENTS

Hereinafter technical solutions according to embodiments of the present disclosure are described clearly and completely in conjunction with the drawings in embodiments of the present disclosure. Apparently, the described embodiments are only a part rather than all of the embodiments of the present disclosure. Any other embodiments obtained by those skilled in the art based on the embodiments of the present disclosure without any creative effort fall within the scope of protection of the present disclosure.

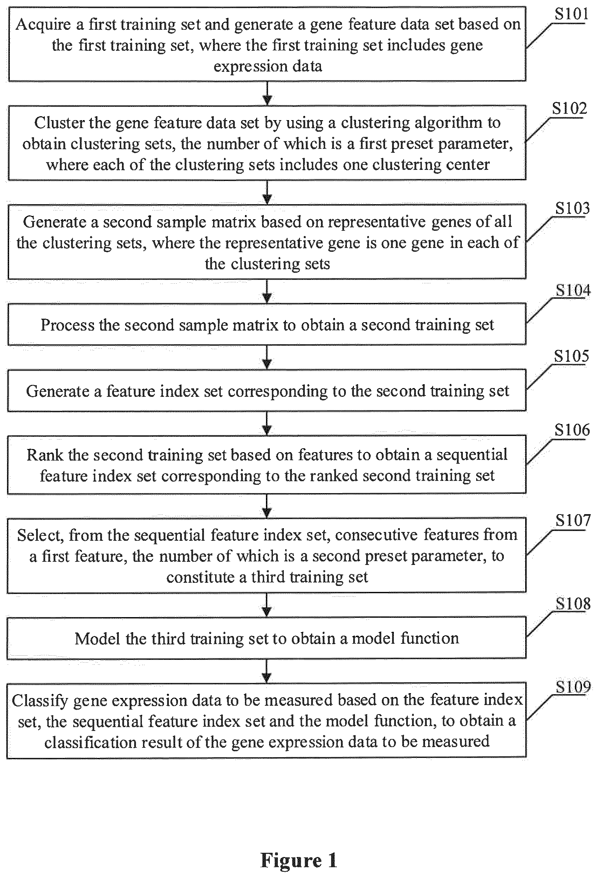

A gene expression data classification method is provided according to an embodiment of the present disclosure. As shown in Figure 1, the gene expression data classification method includes steps S101 to S109.

In step S101, a first training set is acquired, and a gene feature data set is generated based on the first training set. The first training set includes gene expression data.

It should be noted that, the gene expression data in the first training set is acquired through DNA microarray technology.

However, in other embodiments of the present disclosure, the gene expression data may also be acquired through other technology or devices. A method or an apparatus for acquiring the gene expression data is not limited in the present disclosure, which depends on practical conditions.

In an embodiment of the present disclosure, the gene expression data is obtained through the DNA microarray technology to constitute the first training set $\{\mathbf{x}_i, y_i\}_{i=1}^{N}$, where $\mathbf{x}_i$ is the gene expression data in the first training set, $\mathbf{x}_i \in R^D$, $\mathbf{x}_i$ is the gene expression data in the first training set, $y_i$ is a tag of $\mathbf{x}_i$ and is for indicating a type of $\mathbf{x}_i$, $y_i \in \{-1, +1\}$, N represents the total number of samples in the first training set, $D$ represents a dimension of the sample in the first training set, and R represents a set of real numbers. The first training set is preprocessed to generate a first sample matrix $\mathbf{X} = [\mathbf{x}_1, \cdots, \mathbf{x}_N]$. Each column of the first sample matrix constitutes one sample of the first training set. Each row of the first sample matrix, that is, $\mathbf{g}_j = (X_{j\cdot})^T$, $j = 1, \cdots, D$, is selected to constitute the gene feature data set $\{\mathbf{g}_j\}_{j=1}^{D}$, $g_j \in R^N$.

In step S102, the gene feature data set is clustered using a clustering algorithm, to obtain clustering sets, the number of which is a first preset parameter. Each of the clustering sets includes one clustering center.

Each of the clustering sets includes similar gene expression data in the gene feature data set, each of the clustering sets includes one clustering center, and the clustering center of the clustering set is calculated based on all the gene expression data in the clustering set. In an embodiment of the present disclosure, the clustering center of each clustering set is an average value of all the gene expression data in the clustering set, however, which is not limited in the present disclosure, and the clustering center of the clustering set may also be determined in other manner depending on practical conditions.

It should be noted that, an object of clustering the gene feature data set by using the clustering algorithm is to reduce dimensionality of the gene expression data, thereby reducing redundancy among the gene expression data.

In step S103, a second sample matrix is generated based on representative genes of all the clustering sets. The representative gene is one gene in of each of the clustering sets.

In step S104, the second sample matrix is processed to obtain a second training set.

After the representative genes of all the clustering sets are obtained, the second sample matrix is constituted based on the representative genes of all the clustering sets, and each column of the second sample matrix is selected to constitute the second training set.

In step S105, a feature index set corresponding to the second training set is generated.

In step S106, the second training set is ranked based on features to obtain a sequential feature index set corresponding to the ranked second training set.

It should be illustrated that, in an embodiment of the present disclosure, the second training set is ranked based on features using an SVM-RFE algorithm, to obtain the sequential feature index set corresponding to the ranked second training set.

In step S107, consecutive features from a first feature, the number of which is a second preset parameter, are selected from the sequential feature index set to constitute a third training set.

A value of the second preset parameter is smaller than a value of the first preset parameter.

In step S108, the third training set is modeled to obtain a model function.

In an embodiment of the present disclosure, the third training set is modeled through a support vector machine classifier to obtain the model function.

In step S109, gene expression data to be measured is classified based on the feature index set, the sequential feature index set and the model function, to obtain a classification result of the gene expression data to be measured.

It should be noted that, the gene to be measured and the first training set are obtained in a same process of collecting the gene expression data.

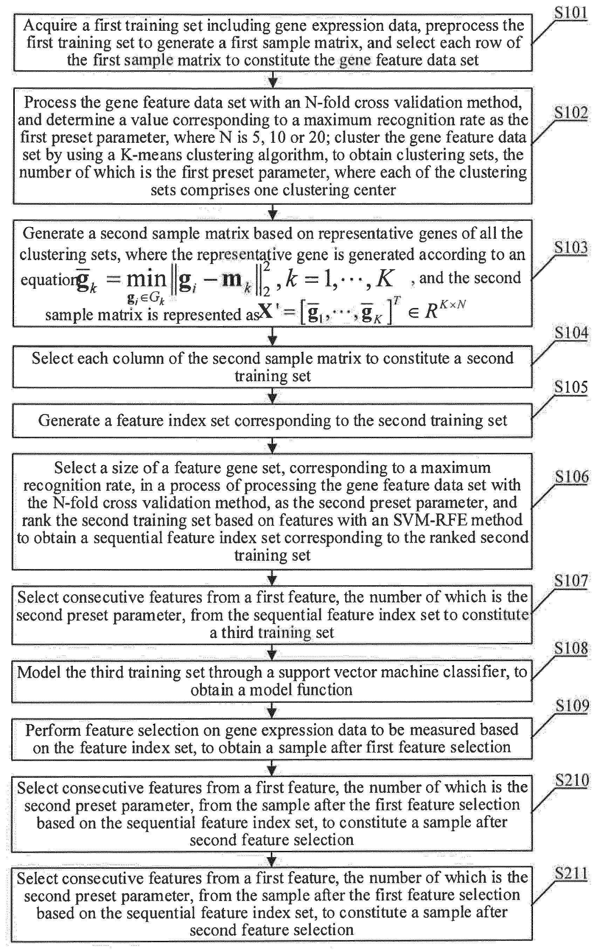

On the basis of the above embodiment, in a preferred embodiment of the present disclosure, as shown in Figure 2, the gene expression data classification method includes steps S201 to S211.

In step S201, a first training set including gene expression data is acquired, the first training set is preprocessed to generate a first sample matrix, and each row of the first sample matrix is selected to constitute the gene feature data set.

In step S202, the gene feature data set is processed with an N-fold cross validation method, and a value corresponding to a maximum recognition rate is determined as a first preset parameter, where N is 5, 10 or 20, and the gene feature data set is clustered using a K-means clustering algorithm to obtain clustering sets, the number of which is the first preset parameter, and each of the clustering sets includes one clustering center.

In the embodiment, setting of the first preset parameter includes:

processing the gene feature data set with the N-fold cross validation method, and determining the value corresponding to the maximum recognition rate as the first preset parameter, where N is 5, 10 or 20. In a preferred embodiment of the present disclosure, N is 10 preferably.

In step S203, a second sample matrix is generated based on representative genes of all the clustering sets.

The representative gene is generated according to an equation $\overline{\mathbf{g}}_k = \min_{\mathbf{g}_i \in G_k} \|\mathbf{g}_i - \mathbf{m}_k\|_2^2$, $k = 1, ..., K$, and the second sample matrix is represented as $X' = [\overline{\mathbf{g}}_1, \cdots, \overline{\mathbf{g}}_k]^T \in R^{K \times N}$.

In which, R represents a set of real numbers, N represents the total number of samples in the first training set, $G_k$ represents a k-th clustering set, $\overline{\mathbf{g}}_k$ represents a representative gene of the k-th clustering set, $\|\|_2$ represents a norm operation, and a subscript represents that a type of the norm is an Euclidean norm, $m_k$ represents a k-th clustering center, K represents the first preset parameter, and $\mathbf{g}_i$ represents the gene expression data in the clustering set.

In step S204, each column of the second sample matrix is selected to constitute a second training set.

In step S205, a feature index set corresponding to the second training set is generated.

In step S206, a size of a feature gene set, corresponding to a maximum recognition rate, in a process of processing the gene feature data set with the N-fold cross validation method, is selected as the second preset parameter, and the second training set is ranked based on features with an SVM-RFE method to obtain a sequential feature index set corresponding to the ranked second training set.

In step S207, consecutive features from a first feature, the number of which is the second preset parameter, are selected from the sequential feature index set to constitute a third training set.

In step S208, the third training set is modeled through a support vector machine classifier, to obtain a model function.

In step S209, feature selection is performed on gene expression data to be measured based on the feature index set, to obtain a sample after first feature selection.

The gene to be measured and the gene expression data of the first training set are collected through the same DNA microarray technology.

In step S210, consecutive features from a first feature, the number of which is the second preset parameter, is selected from the sample after the first feature selection based on the sequential feature index set, to constitute a sample after second feature selection.

In step S211, the sample after the second feature selection is inputted into the model function to obtain an output result of the model function, and a classification result of the gene expression data to be measured is obtained based on the output result.

On the basis of the above embodiments, in an embodiment of the present disclosure, the gene expression data classification method provided according to the embodiment of the present disclosure is tested for a breast cancer data set. The breast cancer data set includes 97 patient samples belonging to two categories. Each of the samples includes 24481 gene expression data. The first training set includes 78 patient samples, in which, 34 patient samples refer to patients (labeled as "relapse") whose cancer cells metastasize during at least 5 years, and the other 44 patient samples refer to patients (labeled as "non-relapse") who are still healthy after at least 5 years from preliminary diagnosis. Correspondingly, gene samples to be measured include 12 "relapse" patient samples and 7 "non-relapse" patient samples.

Testing steps are described as follows.

Gene expression data in the breast cancer data set is acquired through the DNA microarray technology to constitute a first training set $\{\mathbf{x}_i, y_i\}_{i=1}^N$, where $\mathbf{x}_i$ represents the gene expression data in the first training set, $\mathbf{x}_i \in R^D$, $y_i$ is a tag of $\mathbf{x}_i$ and is for indicating a type of $x_i$, $y_i \in \{-1, +1\}$, N represents the total number of samples in the first training set, D represents a dimension of the sample in the first training set, and R represents a set of real numbers, and here N=97 and D=24481.

The first training set is preprocessed to generate a first sample matrix $\mathbf{X} = [\mathbf{x}_1, \cdots, \mathbf{x}_N]$, where each column of the matrix constitutes one sample of the first training set. Each row of the first sample matrix, that is, $\boldsymbol{g}_j = (X_j.)^T$, $j = 1, \cdots, 24481$, is selected to constitute the gene feature data set $\{\mathbf{g}_j\}_{j=1}^{24481}$, $g_j \in R^{97}$.

The number of clustering centers is determined as a first preset parameter K, where K=80 (which is selected through the 10-fold cross validation method). The gene feature data set $\{\mathbf{g}_j\}_{j=1}^{24481}$ is clustered using the K-means clustering algorithm, to obtain 80 clustering centers $m_k$ and 80 clustering sets Gk, k = 1, $\cdots$, K.

One gene is selected from each of the clustering sets as a representative gene of the clustering set, and the representative gene is selected according to an equation $\overline{\mathbf{g}}_k = \min_{\mathbf{g}_i \in G_k} \|\mathbf{g}_i - \mathbf{m}_k\|_2^2$, k = 1, ..., 80, where $\| \|_2$ represents a norm operation, a subscript represents that a type of the norm is Euclidean norm, $G_k$ represents a k-th clustering set, $\overline{\mathbf{g}}_k$ represents a representative gene of the k-th clustering set, and $m_k$ represents a k-th clustering center. A sample matrix $X' = [\overline{\mathbf{g}}_1, \cdots, \overline{\mathbf{g}}_{80}]^T \in R^{80 \times 97}$ is generated, where N represents the total number of training samples in the training set. Each column $\overline{\mathbf{g}}_i$ of the sample matrix X' is selected to function as $\mathbf{x}_i'$ to constitute the second training set $\{\mathbf{x}_i', y_i\}_{i=1}^{97}$, where $\mathbf{x}_i' \in R^{80}$, and a feature index set F $\subset \{1, \cdots, 24481\}, |F| = 80$ corresponding to the second training set is generated.

A value d of the second preset parameter is determined, and the value d (d<80) of the second preset parameter is equal to a size of a feature gene set corresponding to the maximum recognition rate in a process of processing the gene feature data set with the 10-fold cross validation method. The second training set $\{\mathbf{x}_i', y_i\}_{i=1}^N$ is ranked based on features with the SVM-RFE method to obtain a sequential feature index set $F' \subset \{1, \cdots, 24481\}, |F'| = 80$. First d features are selected from the sequential feature index set, to constitute a third training set $\{\mathbf{x}_i'', y_i\}_{i=1}^{97}$, where $\mathbf{x}_i'' \in R^d$ In the embodiment, d=37.

The third training set $\{\mathbf{x}_i'', y_i\}_{i=1}^{97}$ is modeled through the support vector machine classifier, to obtain the model function $f(\mathbf{x}'')$.

The gene expression data (which is the cancer gene expression data in the embodiment) to be measured is denoted as $\mathbf{x}$, where $\mathbf{x} \in R^{24481}$.

Feature selection is performed on the gene expression data $\mathbf{x}$ ($\mathbf{x} \in R^D$) to be measured based on the feature index set F, to obtain a sample $\mathbf{x}'$ ($\mathbf{x}' \in R^K$) after first feature selection.

Consecutive features from a first feature, the number of which is the second preset parameter, are selected from the sample $\mathbf{x}'$ after the first feature selection based on the sequential feature index set $F'$, to constitute a sample

**x"** (**x"** $\in R^d$) after second feature selection.

The sample **x"** after the second feature selection is inputted into the model function $f$(**x"**), to obtain an output result of the model function, and a classification result of the gene expression data to be measured is obtained based on the output result.

The same testing process as described above is applied to the gene expression data classification method according to the embodiment of the present disclosure, the SVM-Recursive Feature Elimination (SVM-RFE) algorithm and the minimal redundancy-maximal relevance + SVM-Recursive Feature Elimination (MRMR+SVM-RFE) algorithm to perform contrast experiments for the same breast cancer data set. 78 training samples are selected randomly for 10 times, and Table 1 shows contrast among best average classification performances of the above three methods.

Table 1 Contrast among best classification performances of SVM-RFE, MRMR+SVM-RFE and gene expression data classification method according to the present disclosure

| Method | The number of reserved features | Positive recognition rate (%) | Negative recognition rate (%) | Average recognition rate (%) | Time (s) |
|---|---|---|---|---|---|
| Gene expression data classification method according to the present disclosure | 37 | 85.71 | 100.00 | 94.74 | 17.175 |
| SVM-RFE | 29 | 57.14 | 100.00 | 84.21 | 4764.271 |
| MRMR+SVM-RFE | 14 | 53.57 | 85.42 | 73.68 | 791.034 |

It can be seen from the contrast in Table 1 that, as compared with the other two algorithms, with the gene expression data classification method according to the embodiment of the present disclosure, various parameters are improved and time consumption is reduced greatly.

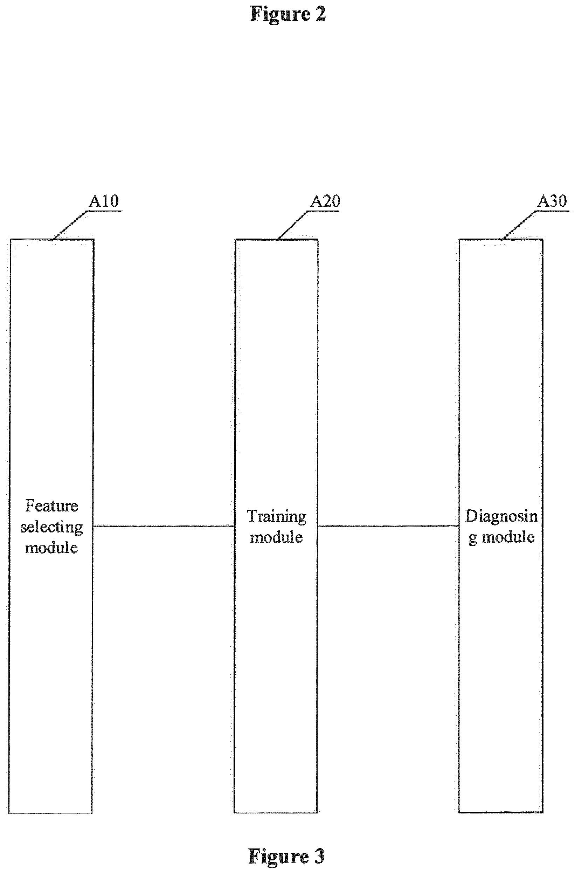

Correspondingly, a gene expression data classification system is further provided according to an embodiment of the present disclosure. As shown in Figure 3, the system includes a feature selecting module A10, a training module A20 and a diagnosing module A30.

The feature selecting module A10 is configured to: acquire a first training set and generate a gene feature data set based on the first training set, where the first training set includes gene expression data; cluster the gene feature data set by using a clustering algorithm, to obtain clustering sets, the number of which is a first preset parameter, where each of the clustering sets includes one clustering center; generate a second sample matrix based on representative genes of all the clustering sets, where the representative gene is one gene in each of the clustering sets; process the second sample matrix to obtain a second training set, and generate a feature index set corresponding to the second training set; rank the second training set based on features to obtain a sequential feature index set corresponding to the ranked second training set; and select, from the sequential feature index set, consecutive features from a first feature, the number of which is a second preset parameter, to constitute a third training set.

The training module A20 is configured to model the third training set to obtain a model function.

The diagnosing module A30 is configured to classify gene expression data to be measured based on the feature index set, the sequential feature index set and the model function, to obtain a classification result of the gene expression data to be measured.

It should be noted that, in the embodiment, the gene expression data to be measured and the first training set are obtained through biological microarray technology in a same collecting process.

With the gene expression data classification system, the gene feature data set is acquired, and then the gene feature data set is clustered using the clustering algorithm to obtain the clustering sets, the number of which is the first preset parameter, and each of the clustering sets includes one clustering center, then, the clustering sets are processed to obtain the second sample matrix, the second training set and the feature index set, to reduce dimensionality of the gene expression data. In this way, redundancy among the gene expression data is reduced, thereby greatly reducing calculation resources and calculation time consumed by the subsequent process of performing feature selection on the second training set. Also, a few calculation resources and a little calculation time are consumed in a case of clustering the gene feature data set by using the clustering algorithm, thereby greatly reducing calculation resources and calculation time consumed by classifying the gene expression data to be measured.

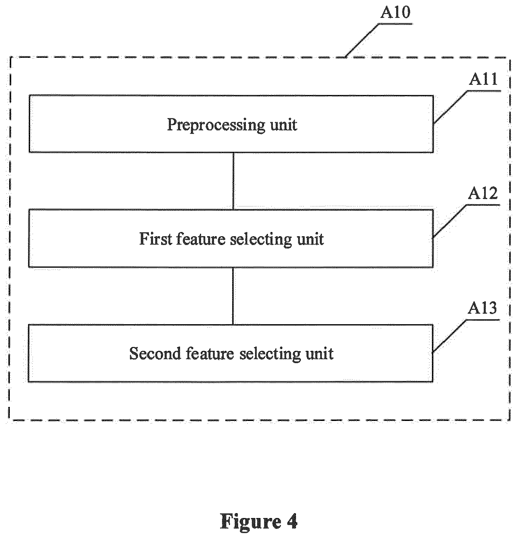

On the basis of the above embodiments, in an embodiment of the present disclosure, as shown in Figure 4, the feature selecting module A10 includes a preprocessing unit A11, a first feature selecting unit A12 and a second feature selecting unit A13.

The preprocessing unit A11 is configured to acquire a first training set of gene samples, preprocess the first training set to generate a first sample matrix, and generate a gene feature data set based on the sample matrix.

The first feature selecting unit A12 is configured to: process the gene feature data set with an N-fold cross validation method, and determine a value corresponding to a maximum recognition rate as the first preset parameter, where N is 5, 10 or 20; cluster the gene feature data set by using a K-means clustering algorithm to obtain clustering sets, the number of which is the first preset parameter, where each of the clustering sets includes one clustering center; select one gene from each of the clustering sets as a representative gene of the clustering set, generate the second sample matrix based on the representative genes of all the clustering sets; and process the second sample matrix to obtain the second training set, and generate the feature index set corresponding to the second training set.

The second feature selecting unit A 13 is configured to rank the second training set based on features to obtain the sequential feature index set, determine the second preset parameter as the number of reserved features, and select, from the sequential feature index set, consecutive features from a first feature, the number of which is the second preset parameter, to constitute the third training set.

It should be noted that, in the embodiment, the preprocessing unit A11 acquires the first training set $\{\mathbf{x}_i, y_i\}_{i=1}^N$ of the gene expression data through the DNA microarray technology, where $\mathbf{x}_i$ represents the gene expression data in the first training set, $\mathbf{x}_i \in R^D$, $y_i$ is a tag of $\mathbf{x}_i$ and is for indicating a type of $\mathbf{x}_i$, $y_i \in \{-1, +1\}$, N represents the total number of samples in the first training set, D represents a dimension of the sample in the first training set, and R represents a set of real numbers. The first training set is preprocessed to generate a first sample matrix $\mathbf{X} = [\mathbf{x}_1, \cdots, \mathbf{x}_N]$, where each column of the matrix constitutes one sample of the first training set. Each row of the first sample matrix, that is, $\mathbf{g}_j = (X_j)^T$, $j = 1, \cdots, D$, is selected to constitute the gene feature data set $\{\mathbf{g}_j\}_{j=1}^D$, $g_i \in R^N$.

In the embodiment, an object of clustering the gene feature data set by using the K-means clustering algorithm is to reduce dimensionality of the gene expression data, so as to reduce redundancy among the gene expression data.

On the basis of the above embodiments, in another embodiment of the present disclosure, the first feature selecting unit A12 is configured to process the gene feature data set with the N-fold cross validation method, and determine the value corresponding to the maximum recognition rate as the first preset parameter K, where N is 5, 10 or 20.

The gene feature data set is clustered using the K-means clustering algorithm, to obtain K clustering centers $m_k$ and K clustering sets $G_k$, $k = 1, \cdots, K$.

One gene is selected from each of the clustering sets as the representative gene of the clustering set. The representative gene is generated according to an equation $\overline{g}_k = \min_{g_i \in G_k} \|g_i - m_k\|_2^2$, $k = 1, ..., K$, where $G_k$ represents a k-th clustering set, $\overline{g}_k$ represents a representative gene of the k-th clustering set, $m_k$ represents a k-th clustering center, and K represents the first preset parameter. A sample matrix $\mathbf{X}' = [\overline{g}_1, \cdots, \overline{g}_K]^T \in R^{K \times N}$ is generated, where N represents the total number of training samples in the training set. Each column of the sample matrix $\mathbf{X}'$ is selected to function as $\mathbf{x}'_i$ to constitute the second training $set\{\mathbf{x}'_i, y_i\}_{i=1}^N$, where $\mathbf{x}'_i \in R^K$, and the feature index set $F \subset \{1, \cdots, D\}, |F| = K$ corresponding to the second training set is generated.

It should be noted that, in a preferred embodiment of the present disclosure, the gene feature data set is processed with a 10-fold cross validation method, and a value corresponding to a maximum recognition rate is determined as the first preset parameter, however, which is not limited in the present disclosure, and depends on practical conditions.

On the basis of the above embodiments, in another embodiment of the present disclosure, the value d (d<K) of the second preset parameter is equal to a size of a feature gene set corresponding to the maximum recognition rate in a process of processing the gene feature data set with the 10-fold cross validation method. In a preferred embodiment of the present disclosure, the second training set $\{\mathbf{x}'_i, y_i\}_{i=1}^N$ is ranked based on features with the SVM-RFE method to obtain the sequential feature index set $F' \subset \{1, \cdots, D\}, |F'| = K$. First d features are selected from the sequential feature index set to constitute the third training set $\{\mathbf{x}''_i, y_i\}_{i=1}^N$, where $\mathbf{x}''_i \in R^d$.

On the basis of the above embodiments, in yet another embodiment of the present disclosure, the third training set is modeled through a support vector machine classifier, to obtain a model function $f(\mathbf{x}''_i)$. However, a method for modeling the third training set is not limited in the present disclosure, and depends on practical conditions.

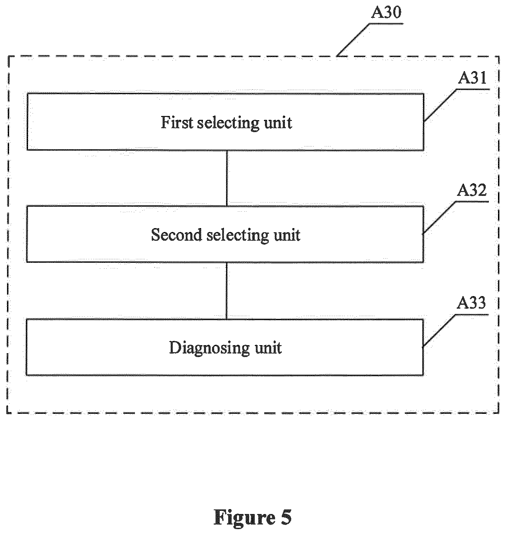

On the basis of the above embodiments, in a specific embodiment of the present disclosure, as shown in Figure 5, the diagnosing module A30 includes a first selecting unit A31, a second selecting unit A32 and a diagnosing unit A33.

The first selecting unit A31 is configured to perform feature selection on gene expression data $\mathbf{x}$ ($\mathbf{x} \in R^D$) to be measured based on the feature index set F, to obtain a sample $\mathbf{x}'$ ($\mathbf{x}' \in R^K$) after first feature selection.

The second selecting unit A32 is configured to select consecutive features from a first feature, the number of which is the second preset parameter, from the sample $\mathbf{x}'$ after the first feature selection based on the sequential feature index set F', to constitute a sample $\mathbf{x}''$ ($\mathbf{x}'' \in R^d$) after second feature selection.

The diagnosing unit A33 is configured to input the sample $\mathbf{x}''$ after the second feature selection into the model function $f(\mathbf{x}'')$ to obtain an output result of the model function, and obtain a classification result of the gene expression data to be measured based on the output result.

In summary, the gene expression data classification method and the gene expression data classification system are provided according to the embodiments of the present disclosure. With the gene expression data classification method, the gene feature data set is acquired, and then the gene feature data set is clustered using the clustering algorithm to obtain clustering sets, the number of which is the first preset parameter, and the clustering sets are processed to obtain the second sample matrix, the second training set and the feature index set, to reduce dimensionality of the gene expression data. In this way, redundancy among the gene expression data is reduced, thereby greatly reducing calculation resources and calculation time consumed in a subsequent process of performing feature selection on the second training set. Also, a few calculation resources and a little calculation time are consumed in a case of clustering the gene feature data set by using the clustering algorithm, therefore, a few calculation resources and a little calculation time are consumed by classifying the gene expression data to be measured with the gene expression data classification method.

The embodiments of the present disclosure are described in a progressive manner, and each embodiment lays emphasis on the difference from other embodiments. Therefore, for the same or similar parts among the embodiments, one may refer to description of other embodiments.

According to the above description of the embodiments of the present disclosure, those skilled in the art can implement or use the present disclosure. Various modifications made to these embodiments are apparent for those skilled in the art, and the general principles defined herein may be implemented in other embodiments without departing from the spirit or scope of the present disclosure. Therefore, the present disclosure is not limited to the embodiments described herein but conforms to a widest scope in accordance with principles and novel features disclosed in the present disclosure.

**Claims**

1. A gene expression data classification method, comprising:

   acquiring a first training set and generating a gene feature data set based on the first training set, wherein the first training set comprises gene expression data;
   clustering the gene feature data set by using a clustering algorithm to obtain clustering sets, the number of which is a first preset parameter, wherein each of the clustering sets comprises one clustering center;
   generating a second sample matrix based on representative genes of all the clustering sets, wherein the representative gene is one gene in each of the clustering sets;
   processing the second sample matrix to obtain a second training set;
   generating a feature index set corresponding to the second training set;
   ranking the second training set based on features to obtain a sequential feature index set corresponding to the ranked second training set;
   selecting, from the sequential feature index set, consecutive features from a first feature, the number of which is a second preset parameter, to constitute a third training set;
   modeling the third training set to obtain a model function; and
   classifying gene expression data to be measured based on the feature index set, the sequential feature index set and the model function, to obtain a classification result of the gene expression data to be measured.

2. The gene expression data classification method according to claim 1, wherein setting of the first preset parameter comprises:

   processing the gene feature data set with an N-fold cross validation method, and determining a value corresponding to a maximum recognition rate as the first preset parameter, where N is 5, 10 or 20.

3. The gene expression data classification method according to claim 1, wherein the clustering the gene feature data set by using the clustering algorithm, to obtain the clustering sets, the number of which being the first preset parameter, and each of the clustering sets comprising one clustering center comprises:

clustering the gene feature data set by using a K-means clustering algorithm, to obtain the clustering sets, the number of which is the first preset parameter, wherein each of the clustering sets comprises one clustering center.

4. The gene expression data classification method according to claim 1, wherein the representative gene is generated according to an equation $\overline{\mathbf{g}}_k = \min_{\mathbf{g}_i \in G_k} \left\| \mathbf{g}_i - \mathbf{m}_k \right\|_2^2$, $k = 1, \ldots, K$,

where $G_k$ represents a k-th clustering set, $\overline{\mathbf{g}}_k$ represents the representative gene of the k-th clustering set, $m_k$ represents a k-th clustering center, K represents the first preset parameter, and $\mathbf{g}_i$ represents the gene expression data of the clustering set; and

wherein the second sample matrix is represented as $\mathbf{X'} = [\overline{\mathbf{g}}_1, \cdots, \overline{\mathbf{g}}_k]^T \in R^{K \times N}$, where R represents a set of real numbers, and N represents the total number of samples in the first training set.

5. The gene expression data classification method according to claim 1, wherein the classifying gene expression data to be measured based on the feature index set, the sequential feature index set and the model function, to obtain the classification result of the gene expression data to be measured comprises:

performing, based on the feature index set, feature selection on the gene expression data to be measured , to obtain a sample after first feature selection;
selecting, from the sample after the first feature selection based on the sequential feature index set, consecutive features from a first feature, the number of which is the second preset parameter, to constitute a sample after second feature selection; and
inputting the sample after the second feature selection into the model function to obtain an output result of the model function, and obtaining, based on the output result, the classification result of the gene expression data to be measured.

6. A gene expression data classification system, comprising:

a feature selecting module configured to: acquire a first training set and generate a gene feature data set based on the first training set, wherein the first training set comprises gene expression data; cluster the gene feature data set by using a clustering algorithm, to obtain clustering sets, the number of which is a first preset parameter, wherein each of the clustering sets comprises one clustering center; generate a second sample matrix based on representative genes of all the clustering sets, wherein the representative gene is one gene in each of the clustering sets; process the second sample matrix to obtain a second training set; generate a feature index set corresponding to the second training set; rank the second training set based on features to obtain a sequential feature index set corresponding to the ranked second training set; and select, from the sequential feature index set, consecutive features from a first feature, the number of which is a second preset parameter, to constitute a third training set;
a training module configured to model the third training set, to obtain a model function; and
a diagnosing module configured to classify gene expression data to be measured based on the feature index set, the sequential feature index set and the model function, to obtain a classification result of the gene expression data to be measured.

7. The gene expression data classification system according to claim 6, wherein the feature selecting module comprises:

a preprocessing unit configured to acquire a first training set of gene samples, preprocess the first training set to generate a first sample matrix, and generate the gene feature data set based on the sample matrix;
a first feature selecting unit configured to: process the gene feature data set with an N-fold cross validation method, and determine a value corresponding to a maximum recognition rate as the first preset parameter, where N is 5, 10 or 20; cluster the gene feature data set by using a K-means clustering algorithm, to obtain clustering sets, the number of which is the first preset parameter, wherein each of the clustering sets comprises one clustering center; select one gene from each of the clustering sets as the representative gene of the clustering set, and generate the second sample matrix based on the representative genes of all the clustering sets; and process the second sample matrix to obtain the second training set, and generate the feature index set corresponding to the second training set; and

a second feature selecting unit configured to rank the second training set based on features, to obtain the sequential feature index set, determine the second preset parameter as the number of reserved features, and select, from the sequential feature index set, consecutive features from a first feature, the number of which is the second preset parameter, to constitute the third training set.

**8.** The gene expression data classification system according to claim 7, wherein the first feature selecting unit is configured to: process the gene feature data set with the N-fold cross validation method, and determine the value corresponding to the maximum recognition rate as the first preset parameter, where N is 5, 10 or 20; cluster the gene feature data set by using the K-means clustering algorithm, to obtain clustering centers and clustering sets, with each of the numbers of the clustering centers and the clustering sets being the first preset parameter; select one gene from each of the clustering sets as the representative gene of the clustering set, and generate the second sample matrix based on the representative genes of all the clustering sets; and select each column $\mathbf{x}_i'$ of the second sample matrix to constitute the second training set $\{\mathbf{x}_i', y_i\}_{i=1}^{N}$, where $\mathbf{x}_i' \in R^K$.

**9.** The gene expression data classification system according to claim 6, wherein the representative gene is generated according to an equation
$$\overline{\mathbf{g}}_k = \min_{\mathbf{g}_i \in G_k} \|\mathbf{g}_i - \mathbf{m}_k\|_2^2 ,$$
$k = 1, ..., K$, where $G_k$ represents a k-th clustering set, $\overline{\mathbf{g}}_k$ represents the representative gene of the k-th clustering set, $m_k$ represents a k-th clustering center, K represents the first preset parameter, and $g_i$ represents the gene expression data of the clustering set; and

wherein the second sample matrix is represented as $\mathbf{X}' = [\overline{\mathbf{g}}_1, \cdots, \overline{\mathbf{g}}_k]^T \in R^{K \times N}$, where R represents a set of real numbers, and N represents the total number of samples in the generated first training set.

**10.** The gene expression data classification system according to claim 6, wherein the diagnosing module comprises:

a first selecting unit configured to perform, based on the feature index set, feature selection on the gene expression data to be measured, to obtain a sample after first feature selection;

a second selecting unit configured to select, from the sample after the first feature selection based on the sequential feature index set, consecutive features from a first feature, the number of which is the second preset parameter, to constitute a sample after second feature selection; and

a diagnosing unit configured to input the sample after the second feature selection into the model function to obtain an output result of the model function, and obtain, based on the output result, the classification result of the gene expression data to be measured.

Acquire a first training set and generate a gene feature data set based on the first training set, where the first training set includes gene expression data — S101

Cluster the gene feature data set by using a clustering algorithm to obtain clustering sets, the number of which is a first preset parameter, where each of the clustering sets includes one clustering center — S102

Generate a second sample matrix based on representative genes of all the clustering sets, where the representative gene is one gene in each of the clustering sets — S103

Process the second sample matrix to obtain a second training set — S104

Generate a feature index set corresponding to the second training set — S105

Rank the second training set based on features to obtain a sequential feature index set corresponding to the ranked second training set — S106

Select, from the sequential feature index set, consecutive features from a first feature, the number of which is a second preset parameter, to constitute a third training set — S107

Model the third training set to obtain a model function — S108

Classify gene expression data to be measured based on the feature index set, the sequential feature index set and the model function, to obtain a classification result of the gene expression data to be measured — S109

**Figure 1**

S101

Acquire a first training set including gene expression data, preprocess the first training set to generate a first sample matrix, and select each row of the first sample matrix to constitute the gene feature data set

S102

Process the gene feature data set with an N-fold cross validation method, and determine a value corresponding to a maximum recognition rate as the first preset parameter, where N is 5, 10 or 20; cluster the gene feature data set by using a K-means clustering algorithm, to obtain clustering sets, the number of which is the first preset parameter, where each of the clustering sets comprises one clustering center

S103

Generate a second sample matrix based on representative genes of all the clustering sets, where the representative gene is generated according to an equation $\overline{\mathbf{g}}_k = \min_{\mathbf{g}_i \in G_k} \left\| \mathbf{g}_i - \mathbf{m}_k \right\|_2^2, k = 1, \cdots, K$, and the second sample matrix is represented as $\mathbf{X}' = \left[ \overline{\mathbf{g}}_1, \cdots, \overline{\mathbf{g}}_K \right]^T \in R^{K \times N}$

S104

Select each column of the second sample matrix to constitute a second training set

S105

Generate a feature index set corresponding to the second training set

S106

Select a size of a feature gene set, corresponding to a maximum recognition rate, in a process of processing the gene feature data set with the N-fold cross validation method, as the second preset parameter, and rank the second training set based on features with an SVM-RFE method to obtain a sequential feature index set corresponding to the ranked second training set

S107

Select consecutive features from a first feature, the number of which is the second preset parameter, from the sequential feature index set to constitute a third training set

S108

Model the third training set through a support vector machine classifier, to obtain a model function

S109

Perform feature selection on gene expression data to be measured based on the feature index set, to obtain a sample after first feature selection

S210

Select consecutive features from a first feature, the number of which is the second preset parameter, from the sample after the first feature selection based on the sequential feature index set, to constitute a sample after second feature selection

S211

Select consecutive features from a first feature, the number of which is the second preset parameter, from the sample after the first feature selection based on the sequential feature index set, to constitute a sample after second feature selection

Figure 2

A10                    A20                    A30

| Feature selecting module | Training module | Diagnosing g module |

Figure 3

A10

A11

Preprocessing unit

A12

First feature selecting unit

A13

Second feature selecting unit

**Figure 4**

A30

A31

First selecting unit

A32

Second selecting unit

A33

Diagnosing unit

**Figure 5**

# INTERNATIONAL SEARCH REPORT

| International application No. |
|---|
| **PCT/CN2016/106255** |

### A.   CLASSIFICATION OF SUBJECT MATTER

G06F 19/24 (2011.01) i

According to International Patent Classification (IPC) or to both national classification and IPC

### B.   FIELDS SEARCHED

Minimum documentation searched (classification system followed by classification symbols)

G06F

Documentation searched other than minimum documentation to the extent that such documents are included in the fields searched

Electronic data base consulted during the international search (name of data base and, where practicable, search terms used)

CNABS, CNTXT, CNKI, DWPI, IEEE: gather, sequencing, gene, cluster, classify, sort, model

### C.   DOCUMENTS CONSIDERED TO BE RELEVANT

| Category* | Citation of document, with indication, where appropriate, of the relevant passages | Relevant to claim No. |
|---|---|---|
| PX | CN 105825081 A (SOOCHOW UNIVERSITY), 03 August 2016 (03.08.2016), claims 1-10 | 1-10 |
| A | CN 102272764 A (ROYAL DUTCH PHILIPS ELECTRONICS LTD.), 07 December 2011 (07.12.2011), the whole document | 1-10 |
| A | CN 104200134 A (BEIJING UNIVERSITY OF TECHNOLOGY), 10 December 2014 (10.12.2014), the whole document | 1-10 |
| A | WO 2001073428 A1 (SHAMIR, R. et al.), 04 October 2001 (04.10.2001), the whole document | 1-10 |

☐ Further documents are listed in the continuation of Box C.        ☒ See patent family annex.

| \*    Special categories of cited documents: | "T"   later document published after the international filing date or priority date and not in conflict with the application but cited to understand the principle or theory underlying the invention |
|---|---|
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 08 February 2017 (08.02.2017) | **15 February 2017 (15.02.2017)** |

| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **TANG, Yuxi** Telephone No.: (86-10) **62411854** |
|---|---|

Form PCT/ISA/210 (second sheet) (July 2009)

**INTERNATIONAL SEARCH REPORT**
Information on patent family members

International application No.

**PCT/CN2016/106255**

| Patent Documents referred in the Report | Publication Date | Patent Family | Publication Date |
|---|---|---|---|
| CN 105825081 A | 03 August 2016 | None | |
| CN 102272764 A | 07 December 2011 | US 2012016826 A1 | 19 January 2012 |
| | | KR 101642270 B1 | 26 July 2016 |
| | | EP 2387758 B1 | 29 May 2013 |
| | | RU 2011133091 A | 20 February 2013 |
| | | US 8712935 B2 | 29 April 2014 |
| | | EP 2207119 A1 | 14 July 2010 |
| | | KR 20110112833 A | 13 October 2011 |
| | | WO 2010079402 A1 | 15 July 2010 |
| | | EP 2387758 A1 | 23 November 2011 |
| | | JP 5674679 B2 | 25 February 2015 |
| | | CN 102272764 B | 28 January 2015 |
| | | JP 2012514783 A | 28 June 2012 |
| | | BR PI0918672 A2 | 23 August 2016 |
| | | IN 201105636 P4 | 30 November 2012 |
| CN 104200134 A | 10 December 2014 | None | |
| WO 2001073428 A1 | 04 October 2001 | None | |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201610246971